# EUROPEAN PATENT APPLICATION

(11) **EP 1 591 133 A1**
(43) Date of publication of application: **02.11.2005**
(21) Application number: 04010309.5
(22) Date of filing: 30.04.2004
(51) Int. Cl.: A61L 27/34, A61L 27/54

(54) **Biologically active implants**

(71) Applicant: SYNTHES, 4436 Oberdorf (CH)
(72) Inventor: Montali, Andrea, 4053 Basel (CH); Schlottig, Falko, 4414 Füllinsdorf (CH)
(74) Representative: Blum, Rudolf Emil

(57) **Abstract**

Described is an osteoinductive implant for for the treatment of pathological changes or conditions of bones and/or intervertebral discs and/or cartilage, in particular implants for regeneration and stabilization of the anatomic features in case of bone fractures caused by external force, osteotomy or pathological changes or conditions, such as for the treatment of the spinal column, for cranial and maxillo-facial applications, dental applications, joint surgery, indications in hollow bones (long bones) and extremities. Said implant comprises at least one secretion inducing substance that induces the secretion of bone and/or cartilage growth stimulating peptides such as growth factors and/or bone morphogenetic proteins, in particular at least one statin.

In a preferred embodiment, the implant is provided with an abrasion-resistant coating comprising a resorbable polymer and at least part of the secretion inducing substance.

## Description

### Field of the invention

The present invention relates to an implant designed to compensate for pathological changes or conditions of bones and/or intervertebral discs and/or cartilage and a method for its production.

### Background of the invention

Implants for the treatment of pathological conditions of the spine and/or locomotor system are known in the prior art. They are intended, for example, to mechanically stabilize a fracture, thus facilitating the healing process or, in the case of endoprosthetic implants, to be permanently bonded to the bone.

WO 98/19699 describes the systemic administration of medications or hormones serving to promote osteosynthesis and thus to accelerate the healing process of the fracture. Examples of suitable means include growth factors such as IGF-I. Such systemic applications, however, can lead to undesirable side effects.

WO 93/20859 describes the fabrication of a thin foil or film consisting of a polylactic acid/polyglycolic acid copolymer containing growth factors. The intent is to e.g. wrap such a foil around fracture-fixation devices prior to their implantation with the intent that the growth factors are released in localized fashion in the area of the fracture. In practice, however, this method is unsuitable since, for instance, a nail wrapped with a foil of that type cannot be inserted in the medulla in a way that the foil, which only loosely envelops the nail, actually reaches the point of its intended healing action.

US 2001/0031274 A1 discloses an implant for pathological changes in the spinal column or locomotor system comprising a varnish-like resorbable polymer coating of a thickness of at most 100 µm including a growth factor. Growth factors, however, have several drawbacks. They are e.g. expensive and fast degraded, such that they have a very limited effective time.

Hitherto statins such as lovastatin and simvastatin that are 3-hydroxy-3-methylglutaryl coenzyme A reductase inhibitors (see e.g. Rossouw et al. , N. Engl. J. Med. 323, 1990) are well known as pharmaceutically active agents that lower the cholesterin. In the last years, they have also caused interest as having the capacity of up-regulating the expression of the BMP-2 gene in osteoblasts (see e.g. US 6,032,887 and US 6,376,476). However, the literature on the bone forming/bone resorption reducing abilities of statins is ambiguous (see e.g. discussion of background art in US 6,022,887 and US 6,376,476 as well as documents cited there).

In light of the foregoing, a need exists for an improved implant that promotes the healing process in pathological changes of the spinal column and locomotor system, especially by furthering osteosynthesis, and thus accelerating the healing of fractures or the integration of an implant, in particular an implant causing none or at most minimal side-effects. It is in particular desired to have an implant, especially a load-bearing implant, that improves the healing process and provides advantages in the production process.

### Summary of the invention

The present invention relates to an implant for treating pathological changes or conditions of bones and/or intervertebral discs and/or cartilage, in particular implants for regeneration and stabilization of the anatomic features in case of bone fractures caused by external force, osteotomy or pathological changes or conditions, such as for the treatment of the spinal column, for cranial and maxillo-facial applications, dental applications, joint surgery, indications in hollow bones (long bones) and extremities. Said implant comprises at least one substance that induces the secretion of bone and/or cartilage growth stimulating peptides such as growth factors and/or bone morphogenetic proteins, in particular at least one statin. In the scope of this invention, a substance that induces the secretion of bone and/or cartilage growth stimulating peptides is also referred to as secretion inducing substance.

The implants, in particular the load-bearing implants, can be made of metals including alloys, inorganic non-metallic materials, polymers or combinations of two or more materials of one of the above mentioned groups of materials or of different groups of materials. Usually, said implants exist as pre-formed bodies. It is, however, also possible to make such implants of liquid or viscous formable materials that cure after implantation and that conserve the form they get at the place of implantation.

Although it is possible to incorporate a secretion inducing substance in the implant body, in the case of implants in the form of pre-formed bodies, the implant preferably has a abrasion-resistant polymer coating comprising said secretion inducing substance, in particular said statin. Abrasion resistant refers to the ability of the polymer coating to withstand abrasion during handling and implantation.

In a preferred embodiment, said polymer coating is resorbable. In general, the coatings of the present invention have an average thickness of 50 µm or less, preferably an average thickness of 25 µm or less, more preferably 20 µm or less, and much desirably 15 µm or less. Usually, the coating has an average thickness of at least about 5 µm. Preferred implants are fracture- and/or osteotomy-fixation device and/or endoprosthetic devices and/or devices used for spinal non-fusion and fusion procedures and/or dental implants and/or implants intended for bone elongating procedures. Also comprised by the present invention are e.g. devices for restauration of cartilage. Examples of such preferred devices include plates, screws, nails, pins, wires, threads, or cages.

Dependent on the intended effect and application, the polymer used for the coating may have a glass transition temperature of at least 37°C or below 37°C. Independent of the glass transition temperature, the polymer in general has a mean molecular weight of 100 kDa or less, whereby for many applications suitable polymers should have a mean molecular weight of at least 20 kDa. A presently preferred molecular weight range is 25 to 35 kDa.

Examples of suitable polymers include poly-α-hydroxy acids, polyglycols, polytyrosine carbonates, starch, gelatins, polysaccharides, polyurethanes, polyesters, polyphosphoesters, polyphosphates, polyphosphonates, polyphosphites, modified proteins (e.g. fibrin, casein), polyorthoesters, trimethylenecarbonates, polydioxanones, polycaprolactones, and cellulose, as well as blends and copolymers thereof. Examples of suitable poly-α-hydroxy acids include polylactides, polyglycolic acids and copolymers and blends thereof, whereby polylactides are presently most preferred.

The coating encompassed by the present invention includes at least one secretion inducing substance, in particular at least one statin. Other pharmaceutically active additives such as antibiotics, growth factors, buffers etc. may be present in addition to the secretion inducing substances, in particular the statins.

The present invention also relates to a method of producing an implant having a usually abrasion-resistant, in particular resorbable, polymer coating of a thickness of usually 50 µm or less. Furthermore, the invention relates to an implant produced by such a method. The method for producing an implant includes the following steps: preparing a dispersion of the optionally resorbable polymer and the secretion inducing substance, in particular the statins, in an appropriate solvent; applying the dispersion on the surface to be coated; and allowing the solvent to evaporate.

The term dispersion refers to any given distribution of the polymer and the statin(s) in an organic solvent. This may be a chemical solution, a purely physical dispersion or any intermediate step, especially including, for instance, colloidal solutions.

Appropriate solvents comprise organic solvents and aqueous media, whereby for polylactides presently organic solvents are preferred.

The application and evaporation processes may occur, for example, at a temperature of between 0 and 30°C, and preferably at about 22°C. Additionally, the evaporation of the solvent may occur, for example, in a gaseous atmosphere substantially saturated with solvent vapor. It has, however, been found that often such saturated atmosphere is not necessary. In an exemplary embodiment, the application of the dispersion and the evaporation of the solvent are repeated two or more times.

The dispersion may be filtered prior to its application. Dependent on the particle size and the purity desired, the filtering may occur through a conventional filter or through a micropore filter with a pore size of 0.45 µm or smaller. The secretion inducing substance(s), in particular the statin(s) may be added together with the polymer or at a later stage.

Suitable solvents include ethyl acetate or chloroform with the dispersion containing, for example, 20 to 300 mg of polymer per ml of solvent.

In the case of liquid or viscous formable "implants", the secretion inducing substance(s), such as statins can be mixed with the curable materials or part thereof. The secretion inducing substances, e.g. the statin can be added impregnated on or incorporated into an e.g. water-soluble filler that dissolves under the conditions present at the implantation site, or they can be mixed with a resorbable polymer.

### Detailed description of preferred embodiments

In one aspect, the present invention relates to an implant for the treatment of pathological changes or conditions of bones and/or intervertebral discs and/or cartilage, in particular implants for regeneration and stabilization of the anatomic features in case of bone fractures caused by external force, osteotomy or pathological changes or conditions, such as for the treatment of the spinal column, for cranial and maxillo-facial applications, dental applications, joint surgery, indications in hollow bones (long bones) and extremities, wherein said implant comprises at least one substance that induces the secretion of bone and/or cartilage growth stimulating peptides such as growth factors and/or bone morphogenetic proteins (secretion inducing substance), in particular at least one statin.

In a preferred embodiment of this aspect, the implant has an abrasion-resistant coating of a preferably resorbable polymer that comprises at least one statin and that usually has a thickness of up to 50 µm. As used herein, the term implant refers to a device, which in the process of a surgical procedure is at least partially introduced inside the body. In an exemplary embodiment the present invention is directed to implants of the type that serve to support a pathologically changed spinal column and/or locomotor system, especially by providing a mechanical reinforcement. The pathological changes may be in the form of fractures, pathological changes of joints and bones. Application of the implants may involve direct contact with, attachment to or insertion in a bone or other part or element of the spinal column or locomotor system.

The term implant is to be understood in the broadest sense of the word since it also includes, for instance, implants which are used for longative or reductive osteotomies, craniotomies, for tumor and sports-injury-related surgery, in dentistry as well as in the case of oral, maxillary and facial dislocations.

The term fracture- and/or osteotomy-fixation device refers to any device that serves to fix, correct and/or mechanically stabilize a fractured bone and/or an osteotomy. Examples thereof include plates, screws, nails, pins, wires, threads, or cages for the spinal column and locomotor system. Usually, fracture fixation devices are removed after the fracture has healed, but in certain circumstances, they may be permanently left in or on the bone or they can be reabsorbed by the organism. Endoprosthetic implants are designed to permanently remain in the body and usually function as substitutes for a natural body part such as a joint, a bone section or a tooth.

Such implants are e.g. fracture- and/or osteotomy-fixation devices and/or endoprosthetic devices, with preferred fracture- and/or osteotomy-fixation devices being selected from the group consisting of plates, screws, nails, pins, wires, threads, and cages.

The implants according to one embodiment of the invention are made of a base material that is chemically and/or physically different from that of the coating. In many cases, the base material may not be resorbable. This implies that under physiological conditions, where the implant is used and for the length of time during which it is typically retained in the body, the base material will not decay, corrode or in any other way change its physiochemical state, or if it does, then only with negligible deterioration of its desired effect. An implant according to one aspect of the invention will in many cases consist of a metal including alloys such as stainless steel or titanium. Alternatively, the implant may consist of a base material which is itself resorbable or bioresorbable.

In accordance with one aspect of the present invention, the implants are provided with an abrasion-resistant coating. By the term abrasion-resistant (see above) also the following characteristics usually are encompassed: The coating bonds with the surface of the base material with enough adhesive strength such that when the implant is implanted, mechanical friction will not abrase or otherwise damage the coating, or at least not to such an extent as to affect or even destroy its physical effect, as described in more detail further below. For example, it is desired that a nail which is provided with such coating can properly be driven into the bone without any significant abrasion of the coating.

It is preferable that the average thickness of the coating is 50 µm or less. For example, spots with a thickness of more than 50 µm, occasioned by fluctuations in the coating process, would be allowed.

The polymer of the coating preferably consists of a resorbable (bioresorbable) polymer. This means that, due to its exposure to the physiological conditions prevailing in the area of the implant, it will progressively degrade over a period of preferably several weeks, months, or years, through molecular breakdown. These molecular separation products and any other metabolites display no or, at worst, only negligible toxicity and the body should be able to metabolize or excrete all or at least most of them.

It is also possible to use compositions of resorbable polymers having different degradation rates. Since many secretion inducing substances, in particular the statins, are readily dissolved in organic solvents, they can homogeneously be incorporated in fast degrading and/or slowly degrading areas to ensure the desired release profile.

Secretion inducing substances, preferably the statins, in particular if used according to the present invention, surprisingly showed several advantages over the hitherto used growth factors. Statins are easily obtainable in great quantities and reproducible quality. They are soluble in preferred solvents and therefore can be homogeneously distributed in large amounts thereby enabling the generation of the optimal release profile. Furthermore, contrary to growth factors, they are not degraded at the intended place of action but only in the liver. This provides the possibility to further improve their effect by enhancing their residence time at the place of activity by e.g. adjusting the pH. If e.g. a polymer is used that decomposes to acidic products, a pH buffering substance can be incorporated in amounts to adjust the pH of the implantation site in view of minimal inflammation of the site and desired residence time of the statins on said site. Furthermore, statins are more stable than growth factors such that sterilization, e.g. by means of gas and/or irradiation, of the coated implant is possible. This is a great advantage since coating in aseptic conditions as preferred for growth factor comprising coatings is not necessary. Furthermore, statins influence the growth factor production process of the cells such that one statin molecule can generate several growth factor molecules, whereby these growth factors - as a further advantage - are identical to the growth factors of the treated individuals.

The statins used are preferably selected from the group comprising atorvastatin, fluvastatin, lovastatin, rovastatin, simvastatin, cerivastatin and mixtures thereof, whereby simvastatin and lovastatin are presently preferred. It has to be understood that the term statin may also refer to a mixture of two or more statins, although usually one statin is sufficient to get the desired activity, life time and release properties.

The statin is usually applied in amounts of 0.5 to 20 % by weight of the coating.

The release time usually is much shorter than the degrading of the polymer. Usually release times of a few hours or days are desired while the degradation of the polymer takes several days, months or even years.

The polymer is preferably selected from the group comprising poly-α-hydroxy acids, polyglycols, polytyrosine carbonates, starch, gelatins, cellulose as well as blends and copolymers containing these components. Particularly preferred among the poly-α-hydroxy acids are the polylactides, polyglycolic acids, and their copolymers and blends. One example of a suitable polylactide is a racemic poly-D,L-lactide, especially one with a molecular weight of about 30 kDa. Such racemic compounds form an amorphous, abrasion-resistant layer on the surface of the implant. The formation of crystalline polymer structures in the coating and therefore an enantiomerically pure lactide should preferably be avoided.

It is preferred that the resorbable polymer coating comprises at least part, preferably all the secretion inducing substance.

The implant, in particular the coating may contain additional pharmaceutically active agents, such as osteoinductive or biocidal or anti-infection substances. Suitable osteoinductive substances include, for example, growth factors as disclosed in US 2001/0031274 A1. However, while growth factors might positively influence the healing process in an early stage, their presence results in a much more critical sterilization process and enhances the costs. Thus, in preferred embodiments, no growth factors are present.

Other additional pharmaceutically active agents are antibiotics such as aminoglycosides. If at all included, they are usually applied in amounts of 5 to 15 % by weight based on the solid content of the coating.

The coating may also contain pH buffering, in particular osteoconductive substances such as a calcium-phosphate buffer, in particular beta-tricalciumphosphate (TCP). This buffer preferably is present in amounts of about 2 to about 5 % by weight. The pH buffering substances often are hardly soluble in the solvents used. Therefore, it is preferred to apply them as fine powders.

It has proved to be advantageous if the coating is not applied all at once but in several steps leading to several, at least two, layers of the resorbable, in particular statin comprising polymer. These layers can have same or different compositions.

Preferred implant materials are metals selected from the group consisting of titanium, titanium alloys, steel, cobalt-chromium alloys, tantalum, magnesium or combinations of two or more materials of the same or different type.

Alternatively, the implant of the present invention comprises resorbable and non-resorbable polymers. Resorbable polymers are in particular polylactides and/or polylactide-co-glycolides. Non-resorbable polymers are e.g. ultra high molecular weight polyethylene (UHMWPE), polyether ether ketones (PEEK) or blends thereof. Optionally, such polymer materials may be present together with the other materials described above or ceramics.

Preferred ceramics of which the implant may be made alone or in combination with polymers and/or metals are e.g. selected from the group comprising Ca-P ceramics, Ca-S ceramics, Si-O ceramics and mixtures thereof.

This invention also relates to a method for producing an implant of the type described above, which may include the steps of:
a) Preparing a dispersion comprising the resorbable polymer and the secretion inducing substance(s), in particular statin(s), in a solvent;
b) applying the dispersion on the surface to be coated; and
c) allowing the solvent to evaporate.

The application of the dispersion and the evaporation of the solvent (steps b) and c)) preferably take place at a temperature of between 0 and 30°C, and more desirably at a room temperature of about 22°C. This so-called cold coating also allows for temperature-sensitive components to be applied on the implant together with the polymer. Applying the dispersion is performed preferably by immersing the implant in the dispersion. Other ways of applying the coating, for example by brushing, spraying, etc. are also possible. In addition to the polymer and the statin(s), the dispersion may also contain the aforementioned further pharmaceutically active agents such as antibiotic(s) and/or buffer(s).

Dependent on the solvent (including solvent mixtures) used, it may be preferable if the solvent is allowed to evaporate in a gas atmosphere essentially saturated with solvent vapor. In this case, the implant that has been provided with the still solvent comprising coating is transferred into a closed space whose atmosphere is highly solvent-saturated. This will result in a slowed down evaporation of the solvent, and consequently, in a uniform, well-adhering abrasion-resistant coating. During solvent-evaporation, dependent on the viscosity of the coating layer after application, the intended evaporation time and the desired uniformity of the thickness of the coating, movement of the implant to compensate for gravitation forces acting on the coating might be desirable. The preferred evaporation time is between 1 minute and 1 hour, or more preferably 5 to 30 minutes, and most desirably about 10 minutes. It is also preferred to apply the coating by incrementally building it up in several thin layers, for example by repeating the application of the dispersion (step b) and the solvent-evaporation processes (step c) two or more times.

The solvents used preferably are popular organic, non-polar or weakly polar solvents. Presently preferred are ethyl acetate or chloroform with ethyl acetate being especially preferred. Ethyl acetate can be evaporated without solvent saturated atmosphere, i.e. in ambient air. Furthermore, statins readily dissolve therein. Prior to its application on the implant, the dispersion contains secretion inducing substances and an amount of preferably 20 to 300 mg, and more desirably 50 to 150 mg polymer per ml of solvent and optionally other constituents such as osteoinductive or biocidal substances.

Prior to the coating step b), the wettability of the implant is preferably improved, e.g. by at least once and preferably once immersing it into pure solvent.

A further aspect of the present invention enabled by the better availability and stability of statins over growth factors, is that they are also suitable to not only be present in a coating but distributed all over the implant. For example in case of a (nearly) totally resorbable implant, statins distributed in the entire implant body may be sufficiently slowly released to provide an effect during the whole healing/implant degradation process. Such implants can be solid or applied as liquid or viscous formable compositions able to cure at the implantation site. In the latter case, for slow release, the statins may be homogeneously distributed within one or several solids, e.g. buffers and/or resorbable polymers that can be added as "fillers" to enhance the viscosity.

As already mentioned, the invention is especially suitable for load bearing implants. It is, however, not limited thereto. The implants can be applied all over the skeleton of humans and animals. Preferred applications are in the regeneration and stabilization of the anatomic status in case of bone fractures and osteotomies involving the hollow bones (long bones) of the extremities, hands and feet, as well as the whole field of oral and maxilla facial surgery and cranial surgery. Further applications are in the scope of the spinal column.

These and other aspects of the present invention may be more fully understood with reference to the following non-limiting examples, which are merely illustrative of presently preferred embodiments of the present invention, and are not to be construed as limiting the invention, the scope of which is defined by the appended claims.

### Example 1: Method for making spinal fusion cage with a coating that induces the secretion of BMP-2.

A solution of 100 mg/ml of poly(D,L-lactide) with an inherent viscosity of 0.5 dl/g in ethyl acetate is prepared. After complete dissolution of the polymer 15 weight-% of simvastatin are added to the solution. The clear solution is subsequently filtered with a 0.2 µm filter and transferred into a cooled vessel.

A polyetheretherketone (PEEK) cage for spinal fusion procedures is dipped into the solution and extracted at a controlled speed. In order to avoid the accumulation of solution in the internal spaces of the implant, the implant is spun around its central axis immediately after extraction from the coating solution. After a drying time of at least two minutes this procedure is repeated. This procedure yields an implant with a coating that has the ability to stimulate the secretion of bone growth factors in osteoblasts. The thickness of the coating is preferably between 5 µm and 10 µm.

The implant obtained by the described method can be used for spinal fusion procedures, bone formation is increased by the release of simvastatin from the coating. Implantation of the implant occurs by the standard surgical procedure used also for uncoated spinal cages.

### Example 2: Method for making an implant for repair of intertrochanteric fractures with a coating that induces the secretion of BMP-2.

A solution of 70 mg/ml of poly(D,L-lactide) with an inherent viscosity of 0.5 dl/g in ethyl acetate is prepared. After complete dissolution of the polymer 10 weight-% of simvastatin are added to the solution. The clear solution is subsequently filtered with a 0.2 µm filter and transferred into a cooled vessel.

A stainless steel implant for intertrochanteric fracture repair (e.g. DHS® plate and DHS® screw by Synthes®) is dipped into the solution and extracted at a controlled speed. After a drying time of at least two minutes this procedure is repeated. This procedure yields an implant with a coating that has the ability to stimulate the secretion of bone growth factors in osteoblasts. The thickness of the coating is preferably between 10 µm and 20 µm.

The implant obtained by the described method can be used for the repair of intertrochanteric fractures, in particular in the elderly, where bone metabolism may be impaired. Bone formation is increased by the release of simvastatin from the coating. Implantation of the implant occurs by the standard surgical procedure used also for uncoated DHS® plates and screws.

### Example 3: Method for making an implant for fixation of cranial segments after a craniotomy with a coating that induces the secretion of BMP-2.

A solution of 100 mg/ml of poly(D,L-lactide) with an inherent viscosity of 0.3 dl/g in ethyl acetate is prepared. After complete dissolution of the polymer 15 weight-% of simvastatin are added to the solution. The clear solution is subsequently filtered with a 0.2 µm filter and transferred into a cooled vessel.

A titanium implant for fixation of cranial segments (e.g. FlapFix™ by Synthes®) is dipped into the solution and extracted at a controlled speed. After a drying time of at least two minutes this procedure is repeated. This procedure yields an implant with a coating that has the ability to stimulate the secretion of bone growth factors in osteoblasts. The thickness of the coating is preferably between 10 µm and 20 µm.

The implant obtained by the described method can be used for the fixation of cranial segments. Bone formation is increased by the release of simvastatin from the coating. Implantation of the implant occurs by the standard surgical procedure.

While various embodiments of the present invention are described above, it should be understood that this invention is not to be limited to the specifically preferred embodiments described herein.

Further, it should be understood that variations and modifications within the spirit and scope of the invention may occur to those skilled in the art to which the invention pertains. Accordingly, all expedient modifications readily attainable by one versed in the art from the disclosure set forth herein that are within the scope and spirit of the present invention are to be included as further embodiments of the present invention. The scope of the present invention is accordingly defined as set forth in the appended claims.

## Claims

1. An implant for the treatment of pathological changes or conditions of bones and/or intervertebral discs and/or cartilage, wherein said implant comprises at least one secretion inducing substance, said secretion inducing substance inducing the secretion of bone and/or cartilage growth stimulating peptides.

2. The implant of claim 1, wherein the implant is a fracture- and/or osteotomy-fixation device and/or an endoprosthetic device and/or a dental implant, in particular a fracture- and/or osteotomy-fixation device selected from the group consisting of plates, screws, nails, pins, wires, threads, and cages.

3. The implant of claim 1 or 2, wherein said secretion inducing substance is a statin.

4. The implant of anyone of claims 1 to 3, wherein said implant comprises a polymer, in particular a resorbable polymer.

5. The implant of claim 4, wherein the polymer is selected from the group consisting of poly-α-hydroxy acids, polyglycols, polytyrosine carbonates, starch, gelatins, polysaccharides, polyurethanes, polyesters, polyphosphoesters, polyphosphates, polyphosphonates, polyphosphites, modified proteins (e.g. fibrin, casein), polyorthoesters, trimethylenecarbonates, polydioxanones, polycaprolactones, cellulose, and blends and copolymers thereof, in particular a polymer that includes poly-α-hydroxy acids that are selected from the group consisting of polylactides, polyglycolic acids, copolymers and blends thereof.

6. The implant of anyone of the preceding claims, said implant comprising metals selected from the group consisting at titanium, titanium alloys, steel, cobalt-chromium alloys, tantalum, magnesium and combinations thereof.

7. The implant of anyone of the preceding claims, said implant comprising ceramics selected from the group consisting of Ca-P ceramics, Ca-S ceramics, Si-O ceramics and mixtures thereof.

8. The implant of anyone of the preceding claims, wherein said implant comprises an abrasion-resistant, resorbable polymer coating, said coating comprising at least part, preferably all the secretion inducing substance.

9. The implant of claim 8, wherein the abrasion-resistant coating has an average thickness of 50 µm or less, in particular 25 µm or less, more preferably 20 µm or less, and much desirably 15 µm or less.

10. The implant of claim 8 or 9, wherein the abrasion-resistant coating has a thickness of at least about 5 µm, preferably a thickness in a range of about 5 to 50 µm, more preferably in a range of about 5 to 25 µm, especially in a range of about 5 to 20 µm, and much desirably in a range of about 5 to 15 µm.

11. The implant of anyone of claims 8 to 10, wherein the statin percentage of the total weight of the solid coating is 0.5 to 20 % by weight.

12. The implant of anyone of the preceding claims, wherein said implant, in particular said abrasion-resistant coating, contains a further pharmaceutically active additive.

13. The implant of claim 12, wherein said further pharmaceutically active additive is or includes an antibiotic.

14. The implant of claim 12 or 13, wherein said further pharmaceutically active additive is or includes a pH buffering substance.

15. A method for making the implant of anyone of claims 8 to 14 comprising:
a) Preparing a dispersion comprising the resorbable polymer and at least one secretion inducing substance, in particular at least one statin, in a solvent;
b) applying the dispersion on the implant surface to be coated; and
c) allowing the solvent to evaporate.

16. The method of claim 15, wherein the wettability of the implant is improved, in particular by at least once and preferably once immersing said implant into pure solvent prior to step b).

17. The method of claims 15 or 16, wherein the evaporation step c) is performed in essentially solvent free atmosphere, preferably ambient air.

18. The method of anyone of claims 15 to 17, wherein ethyl acetate or chloroform is used as the solvent, preferably ethyl acetate.

19. The implant of anyone of claims 1 to 14 obtainable by the method of anyone of claims 15 to 18.
